(19) **Europäisches Patentamt**

**European Patent Office**

**Office européen des brevets**

(11) **EP 1 623 665 A1**

(12) **EUROPEAN PATENT APPLICATION**

(43) Date of publication:
08.02.2006 Bulletin 2006/06

(51) Int Cl.:
*A61B 5/00* (2006.01)    *A61N 1/30* (2006.01)

(21) Application number: **05016767.5**

(22) Date of filing: **02.08.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **04.08.2004 JP 2004228124**
**25.03.2005 JP 2005089180**

(71) Applicant: **SYSMEX CORPORATION**
**Kobe-shi,**
**Hyogo 651-0073 (JP)**

(72) Inventors:
• **Hagino, Kei**
**Chuo-ku**
**Kobe-Shi**
**Hyogo 651-0073 (JP)**
• **Maekawa, Yasunori**
**Chuo-ku**
**Kobe-Shi**
**Hyogo 651-0073 (JP)**

• **Sawa, Kenichi**
**Chuo-ku**
**Kobe-Shi**
**Hyogo 651-0073 (JP)**
• **Okada, Seiki**
**Chuo-ku**
**Kobe-Shi**
**Hyogo 651-0073 (JP)**
• **Sato, Toshiyuki**
**Chuo-ku**
**Kobe-Shi**
**Hyogo 651-0073 (JP)**
• **Asakura, Yoshihiro**
**Chuo-ku**
**Kobe-Shi**
**Hyogo 651-0073 (JP)**

(74) Representative: **HOFFMANN EITLE**
**Patent- und Rechtsanwälte**
**Arabellastrasse 4**
**81925 München (DE)**

(54) **Blood-sugar level measuring device and method**

(57) An analyzer (1) including an electrical information acquiring section (5,6,7,11,12,16,17) for acquiring an electrical information, preferably conductivity, by supplying an electricity to a skin of a subject; an extracting device (2) for extracting a tissue fluid via the skin of the subject; and a controller (10) which calculates a speed at which an analyte is extracted into the extracting device and converts the calculated analyte-extracting speed into a concentration of the analyte included in the tissue fluid in a body of the subject based on the electrical information acquired by the electrical information acquiring section is disclosed. An analyzing method and a blood-sugar level measuring device are also disclosed.

Fig.3

**Description**

FIELD OF THE INVENTION

The present invention relates to an analyzer, an analyzing method and a blood-sugar level measuring device.

BACKGROUND

Conventionally, as a publicly known device and a method for measuring a concentration of a component included in blood and tissue fluid extracted from a subject, for example, a blood-sugar level measuring device and a blood-sugar level measuring method for measuring a concentration of glucose included in blood and tissue fluid extracted from the subject are known.

For example, a method of measuring blood taken from a fingertip by means of a lancet mechanism (for example, see US patent No. 6, 607, 543) using a blood glucose test paper is known. A device for implementing the method is also commercially available.

However, when the above-mentioned device is used, it is necessary to extract blood by sticking a needle into the fingertip of the subject, which places an unpleasant burden on the subject. In particular, the above-mentioned method is tremendously uncomfortable for a severe diabetic patient because he/she must collect blood in the described manner with each meal.

In order to alleviate the unpleasant burden which the subject undergoes, there is a known method called the glucose extracting method using the reverse iontophoresis method in which an electric energy is applied to skin so as to extract the glucose via the skin (for example, see US patent NO. 5,279,543 and the leaflet of PCT No. 96/000110).

Further, a device for measuring the blood-sugar level by extracting the glucose utilizing the reverse iontophoresis method is also commercially available.

However, in the conventional blood-sugar level measuring devices utilizing the reverse iontophoresis method, it is necessary to collect blood using the lancet mechanism or the like approximately once per day, obtain the blood-sugar level from the collected blood using the blood glucose test paper or the like and further perform calibration using the obtained blood-sugar level in order to calculate the blood-sugar level based on an amount of the glucose included in the tissue fluid extracted from the body.

SUMMARY

The scope of the present invention is defined solely by the appended claims, and is not affected to any degree by the statements within this summary.

A main object of the present invention is to provide an analyzer, an analyzing method and a blood-sugar level measuring device not requiring the calibration using the blood-sugar level obtained from the collected blood.

A first aspect of the present invention is an analyzer that comprises an electrical information acquiring section for acquiring an electrical information by supplying an electricity to a skin of a subject; and a controller for acquiring a component value relating to an amount of a predetermined component included in a tissue fluid extracted via the skin of the subject and converting the component value into a concentration of the predetermined component included in the tissue fluid in a body of the subject based on the electrical information acquired by the electrical information acquiring section.

A second aspect of the present invention is an analyzing method that comprises an extracting step for extracting a tissue fluid from a body of a subject via a skin; a component amount acquiring step for acquiring a component value relating to an amount of a predetermined component included in the tissue fluid extracted in the extracting step; an electrical information acquiring step for acquiring an electrical information by supplying an electricity to the skin; and a component concentration acquiring step for converting the component value into a concentration of the predetermined component included in the tissue fluid in the body of the subject based on the electrical information acquired in the electrical information acquiring step.

A third aspect of the present invention is a blood-sugar level measuring device that comprises an electrical information acquiring section for acquiring an electrical information by supplying an electricity to a skin of a subject; and a controller for acquiring a glucose value relating to a glucose amount included in a tissue fluid extracted via the skin of the subject and converting the glucose value into a blood-sugar level of the subject based on the electrical information acquired by the electrical information acquiring section.

BRIEF DESCRIPTION OF THE DRAWINGS

Fig. 1 is a perspective view showing a state in which a blood-sugar level measuring device according to an embodiment of the present invention is placed on the wrist of a subject.

Fig. 2 is a perspective view of an outer appearance of the blood-sugar level measuring device shown in Fig. 1.

Fig. 3 is a block diagram for describing a configuration of the blood-sugar level measuring device shown in Fig. 1.

Figs. 4 through 6 are equivalent circuit diagrams for describing a method of measuring an electric resistance value executed by the blood-sugar level measuring device shown in Fig. 1.

Fig. 7 is a distribution chart showing a relationship between a current density and a transmission factor.

Fig. 8 is a distribution chart showing a relationship between an electric conductivity and a transmission factor.

Fig. 9 is a distribution chart showing a relationship between a transmission factor calculated based on a blood-sugar level measured by another blood-sugar level measuring device and a transmission factor calculated by means of a method recited in the embodiment.

Fig. 10 is a flow chart illustrating blood-sugar level measuring steps using the blood-sugar level measuring device shown in Fig. 1.

Fig. 11 is a perspective view of a micro needle array used before the blood-sugar level measuring device shown in Fig. 1 is placed on the wrist.

Fig. 12 is a timing chart showing magnitudes of a current and a voltage outputted by the blood-sugar level measuring device shown in Fig. 1.

Figs. 13 through 16 are schematic sectional views of a skin in which extraction holes are formed by the micro needle array shown in Fig. 11.

Fig. 17 is a perspective view showing a state in which a blood-sugar level measuring device according to an another embodiment of the present invention is placed on the wrist of the subject.

Fig. 18 is a perspective view of an outer appearance of the blood-sugar level measuring device shown in Fig. 17.

DETAILED DESCRIPTION OF THE PREFERRED EMBODIMENTS

The preferred embodiments of the present invention are described hereinafter with reference to the drawings.

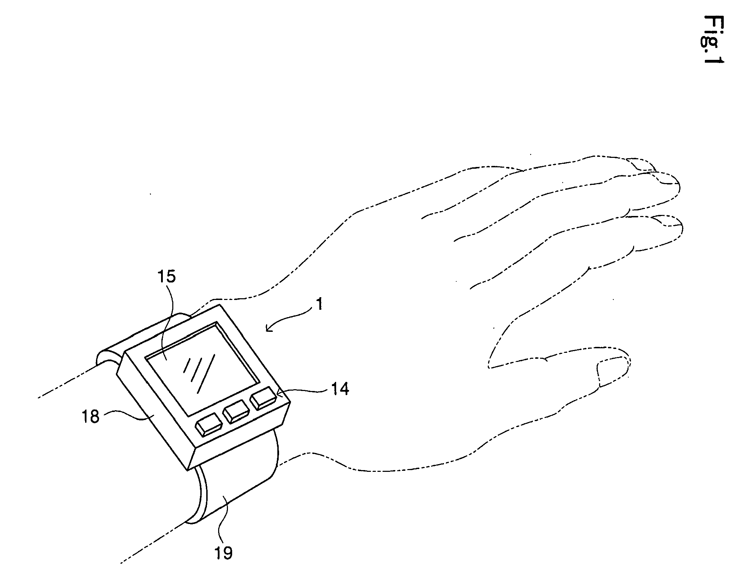

A blood-sugar level measuring device 1, which is placed on the wrist of a subject as shown in Fig. 1, is adapted to extract tissue fluid from a body via the skin thereof and analyze an amount of glucose included in the extracted tissue fluid (extraction tissue fluid) to calculate a blood-sugar level of the subject. The subject is typically a human, however, may be an animal such as a dog, a cat or the like.

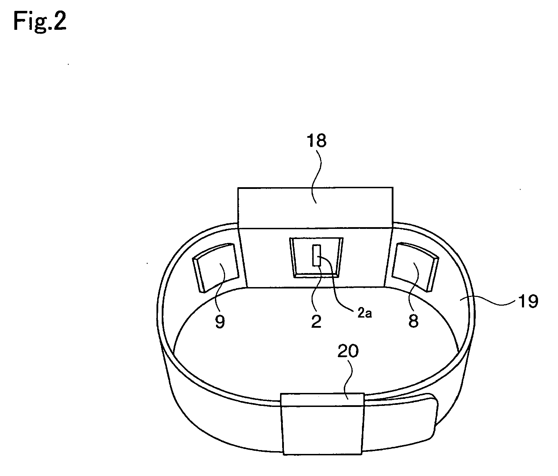

The blood-sugar level measuring device 1, as shown in Figs. 1 and 2, comprises a main body 18, a band 19 for placing the main body 18 on the wrist of the subject in the same manner as placing a wrist watch and a fixing tool 20 for appropriately fastening the band 19 onto the wrist. The main body 18 is fixed to the wrist of the subject by the band 19 and the fixing tool 20. The main body 18 comprises an input unit 14 for inputting an instruction such as an instruction for starting the measurement, a display unit 15 for displaying information such as the blood-sugar level and error information and a chamber 2 provided on a surface of the main body 18 having a contact with the skin. The input unit 14 comprises three key switches. The display unit 15 comprises a liquid crystal display. On a surface of the band 19 having a contact with the skin are provided gelatinous members 8 and 9, which will be described later.

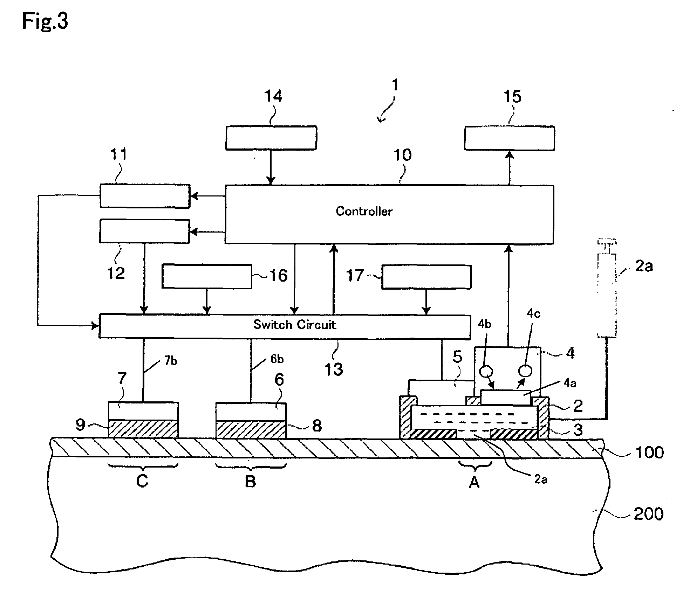

To further describe the configuration referring to Figs. 2 and 3, the blood-sugar level measuring device 1 comprises the chamber 2, a syringe 2a for supplying the chamber 2 with a physiological salt solution 3, a glucose sensor 4 provided adjacent to the chamber 2, an electrode 5, an electrode 6, an electrode 7, the gelatinous member 8 attached to a surface of the electrode 6, the gelatinous member 9 attached to a surface of the electrode 7, a constant current power supply 11, a constant voltage power supply 12, a switch circuit 13, a voltage measuring unit 16, a current measuring unit 17, a controller 10, the input unit 14 and the display unit 15. The electrode 6 is electrically connected to the switch circuit 13 via a cable 6b, and the electrode 7 is electrically connected to the switch circuit 13 via a cable 7b. The electrode 6 is sandwiched between the gelatinous member 8 and the band 19, while the electrode 7 is sandwiched between the gelatinous member 9 and the band 19.

The chamber 2 is configured to contain a liquid therein, and is provided so as to retain the tissue fluid extracted via the skin in the contained liquid.

The chamber 2 has an opening on the skin side and an another opening on the glucose-sensor-4 side opposing to the skin side. The opening of the chamber 2 on the glucose-sensor-4 side is sealed with the glucose sensor 4 and the electrode 5. The opening on the skin side (denoted by a reference 2a in Fig. 2) is provided so as to make the physiological salt solution contact the skin.

The chamber 2 is in a dry state before the blood-sugar level measuring device 1 is used, and the physiological salt solution 3 is supplied from the syringe 2a to the chamber 2 when the blood-sugar level measuring device 1 is used.

The glucose sensor 4 comprises a sensor member 4a disposed so as to contact the physiological salt solution contained in the chamber 2, a light source 4b for irradiating a light on the sensor member 4a and an optical detector 4c for detecting the light irradiated from the light source 4b via the sensor member 4a.

A surface of the sensor member 4a on the chamber-2 side is coated with a color producing pigment reacting to an active oxygen generated from the presence of the glucose.

Therefore, an intensity of the light detected by the optical detector 4c is variable depending on the amount of the glucose retained in the physiological salt solution. The optical detector 4c outputs a signal in compliance with the intensity of the detected light to the controller 10 in response to the detection of the light.

As the glucose sensor 4, for example, a glucose sensor, a semiconductor laser and a photo diode recited in US Patent Publication No. 2003-225322 can be used.

As materials used for the electrodes 5, 6 and 7, carbon or silver chloride can be used.

As the gelatinous members 8 and 9, an unwoven fabric to which polyacrylic acid is attached can be used.

The constant current power supply 11 is a power supply whose magnitude of output current is constant. The constant voltage power supply 12 is a power supply whose magnitude of output voltage is constant. The constant current power supply 11 comprises a transistor constant current circuit combined with a battery incorporated therein. The constant voltage power supply 12 comprises a transistor constant voltage circuit combined with a battery incorporated therein.

The switch circuit 13 is composed of a circuit in which switching elements are combined and changes output destinations of the power supplies 11 and 12. More specifically, the switch circuit 13 switches to and from a circuit in which the current outputted from the constant current power supply 11 circulates through the electrode 7, gelatinous member 9, skin of the subject, gelatinous member 8, electrode 6 and constant current power supply 11, a circuit in which the current outputted from the constant current power supply 11 circulates through the electrode 7, gelatinous member 9, skin of the subject, physiological salt solution supplied to the chamber 2, electrode 5, and constant current power supply 11, and a circuit in which the current outputted from the constant current power supply 11 circulates through the electrode 6, gelatinous member 8, skin of the subject, physiological salt solution supplied to the chamber 2, electrode 5 and constant current power supply 11.

The switch circuit 13 further switches to and from a circuit in which the current outputted from the constant voltage power supply 12 circulates through the electrode 7, gelatinous member 9, skin of the subject, gelatinous member 8, electrode 6 and constant voltage power supply 12, a circuit in which the current outputted from the constant voltage power supply 12 circulates through the electrode 7, gelatinous member 9, skin of the subject, physiological salt solution supplied to the chamber 2, electrode 5, and constant voltage power supply 12, and a circuit in which the current outputted from the constant voltage power supply 12 circulates through the electrode 6, gelatinous member 8, skin of the subject, physiological salt solution supplied to the chamber 2, electrode 5 and constant voltage power supply 12.

The voltage measuring unit 16 measures a magnitude of the voltage outputted from the constant current power supply 11. The current measuring unit 17 measures a magnitude of the current outputted from the constant voltage power supply 12. The magnitude of the voltage outputted from the constant current power supply 11 is equal to a magnitude of the voltage applied to the skin, while the magnitude of the current outputted from the constant voltage power supply 12 is equal to a magnitude of the current running through the skin.

The controller 10 is composed of a microcomputer including CPU, ROM, RAM and the like. The controller 10 controls the operations of the glucose sensor 4, constant current power supply 11, constant voltage power supply 12 and switch circuit 13. Further, the controller 10 calculates a speed at which the glucose is extracted into the chamber 2 based on the output of the optical detector 4c, and converts the calculated glucose-extracting speed into the blood-sugar level based on the outputs of the voltage measuring unit 16 and the current measuring unit 17.

When the blood-sugar level measuring device 1 is used, the subject places the main body 18 on the wrist, and fastens the band 19 onto the wrist to fix the blood-sugar level measuring device 1 thereto using the fixing tool 20. Thereby, the gelatinous members 8 and 9 and the chamber 2 are made to closely contact an epidermis 100 (Fig. 3), and the opening 2a of the chamber 2 and the gelatinous members 8 and 9 are respectively disposed at a portion A and portions B and C. The portion A corresponds to a portion of the epidermis 100 having contact with the physiological salt solution 3 supplied into the chamber 2. The portion B corresponds to a portion of the epidermis 100 having contact with the gelatinous member 8. The portion C corresponds to a portion of the epidermis 100 in contact with the gelatinous member 9.

Below is described a principle by which the blood-sugar level measuring device 1 (controller 10) converts the glucose-extracting speed into the blood-sugar level based on the outputs of the voltage measuring unit 16 and the current measuring unit 17.

In general, it is known that a relationship among a glucose-extracting speed J of the glucose extracted from the body via the skin, a transmission factor P of the glucose with respect to the skin, a surface area S of a portion of the epidermis through which the glucose is transmitted and a concentration C of the glucose included in the tissue fluid in the body of the subject is represented by the following arithmetic expression (1) based on the diffusion principle.

$$J = S \times C \times P \dots \qquad (1)$$

When the arithmetic expression (1) is changed, the glucose concentration C can be represented by the following arithmetic expression (2).

$$C = J / (S \times P) \quad \dots \qquad (2)$$

The tissue fluid is a body fluid including a fluid exuded from a blood capillary. The glucose-extracting speed J can be calculated by dividing the glucose amount in the chamber 2 after a predetermined time (T) has passed since the current supply starts in order to collect the glucose in the chamber 2 by the predetermined time (T). The glucose amount in the chamber 2 can be obtained by means of the glucose sensor 4.

It can be deemed that the concentration C of the glucose included in the tissue fluid (internal tissue fluid) existed in the body of the subject is equal to a concentration of the glucose included in blood existed in the body of the subject, that is the blood-sugar level.

For example, "Continuous Blood-Sugar Level Measuring Device" in the clinical test 39 (8): 894-897, 1995 by Makoto Kikuchi recites that the blood-sugar level corresponds to the concentration of the glucose included in the tissue fluid in the body of the subject.

When a value of the transmission factor P in the arithmetic expression (2) is obtained, the blood-sugar level (hereinafter, the blood-sugar level can be referred to as blood-sugar level C because the glucose concentration C and the blood-sugar level are equal as described above) can be calculated.

Here, the inventors of the present invention examined a method of obtaining the transmission factor P without collecting blood and found out that the transmission factor P was represented by a function of an electric conductivity (conductance) k (that is an inverse number of the electric resistance value) in the glucose-extracting portion of the skin and a magnitude of the current used for extracting the glucose.

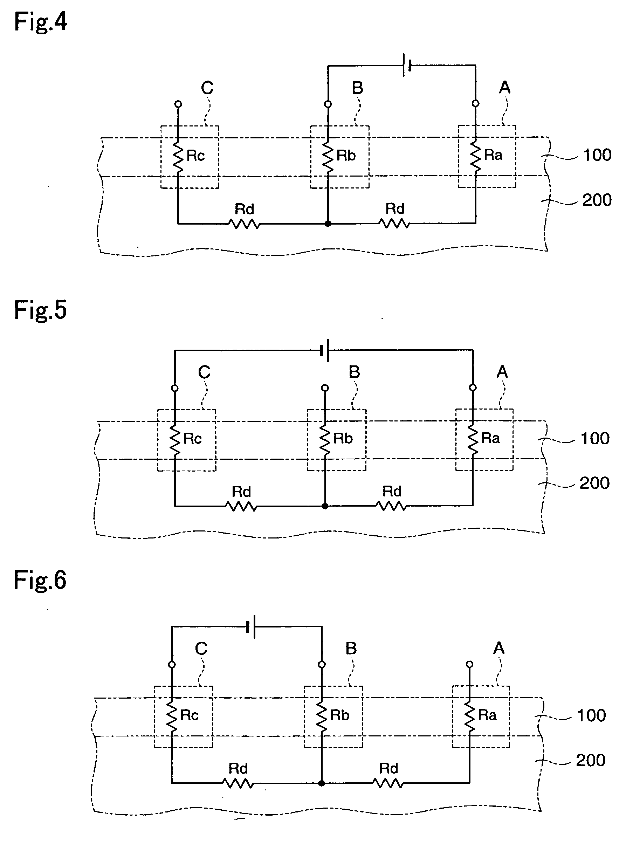

Figs. 4 through 6 are equivalent circuit diagrams for describing a method of obtaining the electric conductivity k in the portion A. In these drawings, reference symbols A, B and C respectively denotes the portions A, B and C shown in Fig. 3. Further, in these drawings, a reference symbol 100 denotes the epidermis, while a reference symbol 200 denotes a tissue disposed closer to the inside of the body than the epidermis 100.

As shown in Fig. 4, a direct current having a certain magnitude (constant current) is applied to between the portions A and B so as to measure a voltage value between the portions A and B. Next, the voltage value between the portions A and B is divided by the magnitude of the constant current applied to between the portions A and B so that an electric resistance value Rab is calculated. Here, the Rab is a sum of an electric resistance value Ra of the portion A of the epidermis, an electric resistance value Rb of the portion B of the epidermis and an electric resistance value Rd between the portions A and B in the tissue 200. The voltage value between the portions A and B may be an average value of the voltage values during a period when the current is applied to between the portions A and B or a voltage value at a predetermined timing during the period when the current is applied.

Further, as shown in Fig. 5, the direct current having the certain magnitude (constant current) is applied to between the portions A and C so as to measure a voltage value between the portions A and C. Next, the voltage value between the portions A and C is divided by the magnitude of the constant current applied to between the portions A and C so that the electric resistance value Rab is calculated. Here, the Rab is a sum of the electric resistance value Ra of the portion A of the epidermis, the electric resistance value Rb of the portion B of the epidermis and an electric resistance value 2Rd between the portions A and C in the tissue 200. The voltage value between the portions A and C may be an average value of the voltage values during a period when the current is applied to between the portions A and C or a voltage value at a predetermined timing during the period when the current is applied.

Further, as shown in Fig. 6, the direct current having the certain magnitude (constant current) is applied to between the portions B and C so as to measure a voltage value between the portions B and C. Next, the voltage value between the portions B and C is divided by the magnitude of the constant current applied to between the portions B and C so that the electric resistance value Rab is calculated. The Rab is a sum of the electric resistance value Ra of the portion A of the epidermis, the electric resistance value Rb of the portion B of the epidermis and the electric resistance value Rd between the portions B and C in the tissue 200. The voltage value between the portions B and C may be an average value of the voltage values during a period when the current is applied to between the portions B and C or a voltage value at a predetermined timing during the period when the current is applied.

Here, the electric resistance in the tissue 200 is sufficiently smaller than the electric resistance of the epidermis 100. Therefore, Rd<<Ra+Rb and 2Rd<<Ra+Rb, as a result of which the above-mentioned relationships are represented by the following arithmetic expressions.

$$Rab = Ra + Rb + Rd = Ra + Rb \dots \quad (3)$$

$$Rac = Ra + Rc + 2Rd = Ra + Rc \quad \dots \quad (4)$$

$$Rbc = Rb + Rc + Rd = Rb + Rc \dots \quad (5)$$

From the arithmetic expressions (3), (4) and (5), the electric resistance value Ra of the portion A of the epidermis is obtained as follows.

$$Ra = (Rab + Rac - Rbc) / 2 \quad \dots \quad (6)$$

Therefore, the electric conductivity k of the portion A of the epidermis is calculated as follows.

$$k = 1 / Ra \quad \dots \quad (7)$$

As another possible method of calculating the electric resistance Rab, a voltage having a certain magnitude is applied to between the portions A and B by the constant voltage power supply, a magnitude of the current outputted from the constant voltage power supply during the application of the voltage is measured, and the value of the voltage outputted from the constant voltage power supply is divided by the obtained current value. The magnitude of the current outputted from the constant voltage power supply during the application of the voltage may be an average value of the current magnitudes of during a period when the voltage is applied to between the portions A and C or a magnitude of the current at a predetermined timing during the period when the voltage is applied.

In the same manner, as another possible method of calculating the electric resistance Rac, the voltage having the certain magnitude is applied to between the portions A and C by the constant voltage power supply, a magnitude of the current outputted from the constant voltage power supply during the application of the voltage is measured, and the value of the voltage outputted from the constant voltage power supply is divided by the obtained current value. As another possible method of calculating the electric resistance Rbc, the voltage having the certain magnitude is applied to between the portions B and C by the constant voltage power supply, a magnitude of the current outputted from the constant voltage power supply during the application of the voltage is measured, and the value of the voltage outputted from the constant voltage power supply is divided by the obtained current value. The magnitude of the current outputted from the constant voltage power supply during the application of the voltage may be the average value of the current magnitudes during the period when the voltage is applied to between the portions A and C (B and C) or the magnitude of the current at the predetermined timing during the application of the voltage.

Next, a relationship between the electric conductivity k and the transmission factor P is obtained through an experiment.

First, as described above, the direct current (constant current) having the certain magnitude is applied to between the portions A and B, between the portions A and C and between the portions B and C (see Figs. 4 through 6) of the subject, and the respective voltage values are measured. Then, the electric conductivity k at the portion A is obtained from the arithmetic expressions (6) and (7).

Further, the chamber 2 is disposed on the portion A of the same subject and the physiological salt solution is supplied to the chamber 2 so that the glucose is extracted without the application of the current to the skin (generally called passive-diffusion extraction). Then, the glucose-extracting speed J in the extraction is measured using the glucose sensor 4. The glucose-extracting speed J is obtained by dividing the glucose amount after the predetermined time (T) has passed since the supply of the physiological salt solution by the predetermined time (T).

Further, blood is collected from the same subject by another blood-sugar level measuring device (for example, "Nipro Free style" manufactured by NIPRO CORPORATION) so that a blood-sugar level C' is measured.

Here, the transmission factor P is represented by $P = J / (S \times C')$ in the same manner as in the arithmetic expression (1). Therefore, when the glucose-extracting speed J and the blood-sugar level C' are assigned to the arithmetic expression, the transmission factor P can be obtained.

Further, the electric conductivity per unit area, that is an electric conductivity k' , can be obtained by dividing the electric conductivity k by a surface area S of the portion A.

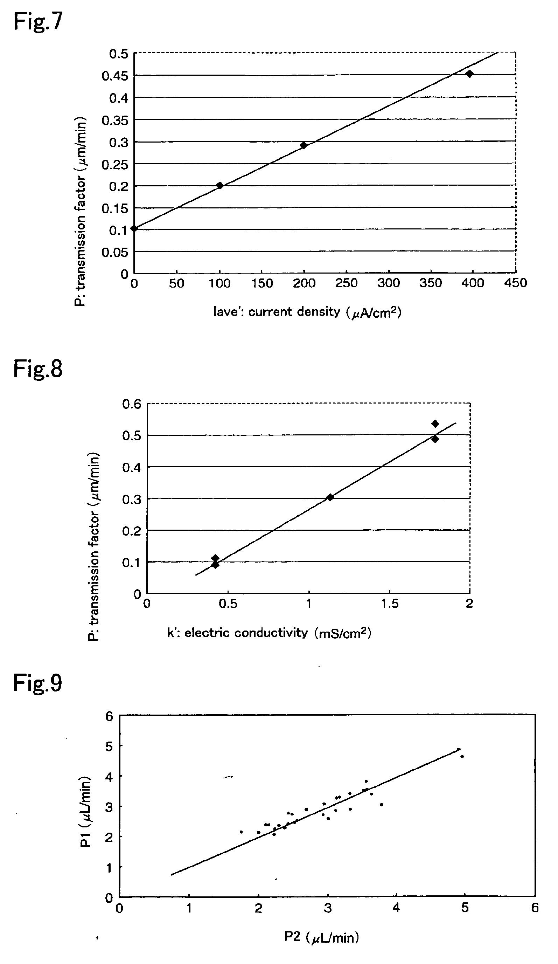

Points corresponding to the electric conductivity k' and the transmission factor P thus obtained are plotted in a distribution chart (see Fig. 8) in which the electric conductivity k' constitutes a horizontal axis and the transmission factor P constitutes a vertical axis.

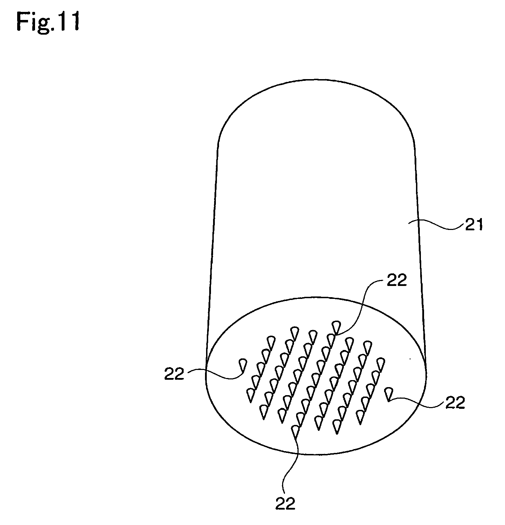

Then, the portion A of the epidermis is stung with micro needles of a micro needle array 21 shown in Fig. 11 (described later) so that the state of the portion A, that is the transmission factor P, is changed. In the above-mentioned manner, the electric conductivity k' and the transmission factor P in a plurality of states of the portion A are calculated, and a result of the calculation is plotted in the afore-mentioned distribution chart in which the electric conductivity k' constitutes the horizontal axis and the transmission factor P constitutes the vertical axis. As a result, the distribution chart of Fig. 8 can be obtained.

The distribution chart shows that the electric conductivity k' and the transmission factor P are in a proportional relationship.

Based on a matter that the electric conductivity k' and the transmission factor P are in the proportional relationship and k' = k / S, the transmission factor P in the case of extracting the glucose by means of the passive diffusion is represented by the following arithmetic expression.

$$S \cdot P = ak + b' \qquad \ldots (8)$$

(a and b' are constants, k is the electric conductivity)

Next, a relationship between an average current Iave running through the portion A of the epidermis and the transmission factor P is experimentally obtained. In the present embodiment, it is assumed that the magnitude of the current outputted from the constant voltage power supply and the magnitude of the current applied to the portion A are equal, however, the magnitude of the current flow in the portion A may be obtained by correcting the magnitude of the current outputted from the constant voltage power supply.

The constant voltage is applied to the skin by the constant voltage power supply in the state in which the chamber 2 is placed on the portion A of the subject and the physiological salt solution is supplied to the chamber 2 so that the tissue fluid including the glucose is extracted in the chamber 2. Then, the average value of the magnitudes of the currents outputted from the constant voltage power supply is calculated during the application of the constant voltage by the constant voltage power supply, and the calculated average value is divided by the surface area S of the portion A of the epidermis so that a current density Iave' is calculated.

Further, the glucose-extracting speed J during the application of the constant voltage in order to extract the tissue fluid including the glucose is measured by means of the glucose sensor 4. The glucose-extracting speed J can be obtained by dividing the glucose amount after the predetermined time (T) has passed from the start of the voltage application by the predetermined time (T).

Further, blood is collected from the same subject by the another blood-sugar level measuring device (for example, "Nipro Free style" manufactured by NIPRO CORPORATION) so that the blood-sugar level C' is measured.

Here, as described, the transmission factor P is represented by P = J / (S×C') as described. Therefore, when the glucose-extracting speed J and the blood-sugar level C' are assigned to the arithmetic expression, the transmission factor P can be obtained.

Next, the output voltage of the constant voltage power supply is changed step by step. Then, the current density Iave' and the transmission factor P with respect to a plurality of output voltages are calculated in the same manner as described before, and a result of the calculation is plotted in a distribution chart in which the current density Iave' constitutes a horizontal axis and the transmission factor P constitutes a vertical factor so as to obtain a distribution chart shown in Fig. 7. It is learnt from the distribution chart that the current density Iave' and the transmission factor P are in a proportional relationship.

Based on a matter that the current density Iave' and the transmission factor P are in the proportional relationship and Iave' = Iave / S the transmission factor P is represented by the following arithmetic expression.

$$S \cdot P = cIave + b'' \qquad \ldots \qquad (9)$$

(a and b" are constants)

Therefore, based on the arithmetic expressions (8) and (9), when the glucose is extracted into the chamber 2 through the application of the voltage to the skin (current supply), the transmission factor P is represented by the following

arithmetic expression.

$$S \cdot P = ak + b + c \ Iave \quad \dots \quad (10)$$

(a and b are constants)

Through the above-mentioned experiment, examples of the constants a, b and c are obtained as follows.

$$a = 3.6 \mu L/min \cdot mS$$

$$b = -0.36 \mu L/min$$

$$c = 0.011 \mu L/min \cdot \mu A$$

When these values are assigned to the arithmetic expression (10), the following is obtained.

$$S \cdot P = 3.6k - 0.36 + 0.011 \ Iave \dots \quad (11)$$

Therefore, based on the arithmetic expressions (2) and (11), the blood-sugar level C is represented by the following arithmetic expression.

$$C = J / (3.6k - 0.36 + 0.011 \ Iave) \ \dots \ (12)$$

In the case of Rab = 5k$\Omega$, Rbc = 6k$\Omega$, and Rac = 7k $\Omega$,
k = 0.33 [mS] is obtained from the arithmetic expressions (6) and (7), and
in the case of Iave = 150$\mu$A and J = 30ng/min, the blood-sugar level C is calculated from the arithmetic expression (12) as follows.

$$C = 30/(3.6 \times 0.33 - 0.36 + 0.011 \times 150) = 12.0 \ [ng/\mu L] = 120$$

$$(mg/dL) \quad \dots \quad (13)$$

Fig. 9 shows a correlation with respect to 30 subjects between a transmission factor P1 calculated using the arithmetic expression (P = J / (S $\times$ C')) based on the blood-sugar level C' obtained by the another blood-sugar level measuring device ("Nipro Free Style" manufactured by NIPRO CORPORATION) and the glucose-extracting speed J obtained by the glucose sensor 4 and a transmission factor P2 obtained by the method according to the present embodiment based on the arithmetic expression (11)

It is learnt from Fig. 9 that a close correlation is observed between P1 and P2.

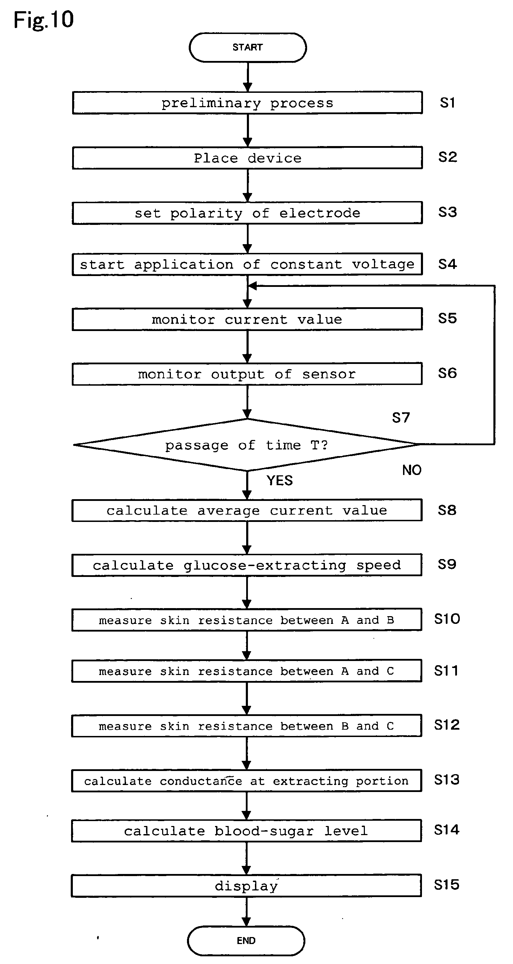

Next is described steps of the blood-sugar measurement using the blood-sugar level measuring device 1 using the principle so far described referring to a flow chart of Fig. 10.

The controller 10 of the blood-sugar level measuring device 1 is previously provided with a program for calculating the blood-sugar level using the arithmetic expressions (6), (7) and (12).

When the blood-sugar level is measured, the following preliminary process is carried out prior to the placing of the device 1 on the wrist (Step S1). As shown in Fig. 11, in the micro needle array 21 used in the preliminary process, 49 needles each having the height of 0. 4mm from an edge surface of needle array 21and the base thickness of 0. 24mm are equally spaced in the area of 10mm$\times$10mm in a protruding manner. The subject stings a part of the epidermis 100 including the portions A, B and C shown in Fig. 3 using the micro needle array 21 a plurality of times (for example, three times) (Step S1). As a result, a plurality of micro holes (extraction holes) is formed in the respective portions of the epidermis so as to improve the transmission factor of the glucose (tissue fluid).

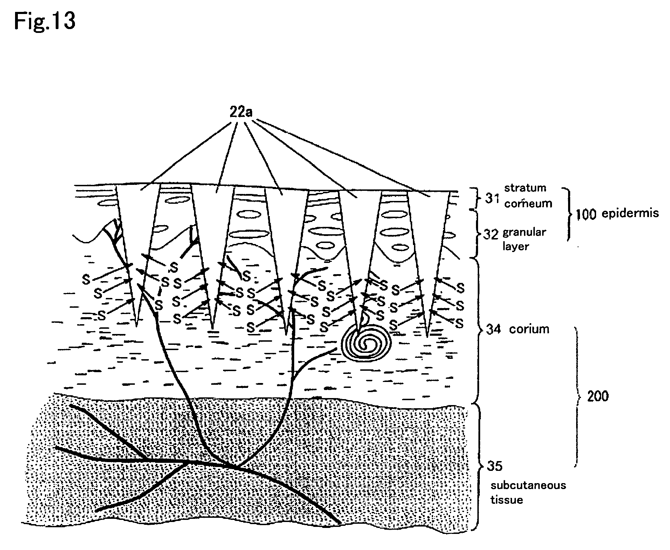

As shown in Fig. 13, a plurality of extraction holes 22a formed in the portions A, B and C in the Step S1 penetrates through a stratum corneum 31 and a granular layer 32 and reaches a approximately intermediate part of a corium 34, however failing to reach a subcutaneous tissue 35. Further, in the extraction holes 22a, a diameter is the largest in the skin surface, while becoming smaller toward the subcutaneous tissue 35. The epidermis 100 comprises the stratum corneum 31, the granular layer 32 and the like.

As a result of the formation of the extraction holes 22a in the Step S1, the tissue fluid saturating the corium 34 is exuded into the extraction holes 22a as shown in arrow S. The tissue fluid includes the glucose.

After the removal of the micro needles 22 from the skin, in Step S2, the blood-sugar level measuring device 1 is placed on the wrist of the subject as shown in Fig. 1. When the blood-sugar level measuring device 1 is thereafter fixed by the band 19 and the fixing tool 20, the chamber 2, gelatinous member 8 and gelatinous member 9 are respectively brought into a close contact with the portions A, B and C of the epidermis 100 as shown in Fig. 3. The subject operates the syringe 2a to supply the physiological salt solution 3 into the chamber 2 from the syringe 2a.

Thereby, the blood-sugar level measuring device 1 is in the state in which the chamber 2 is placed on the portion A and the physiological salt solution 3 is in contact with the portion A as shown in Fig. 3.

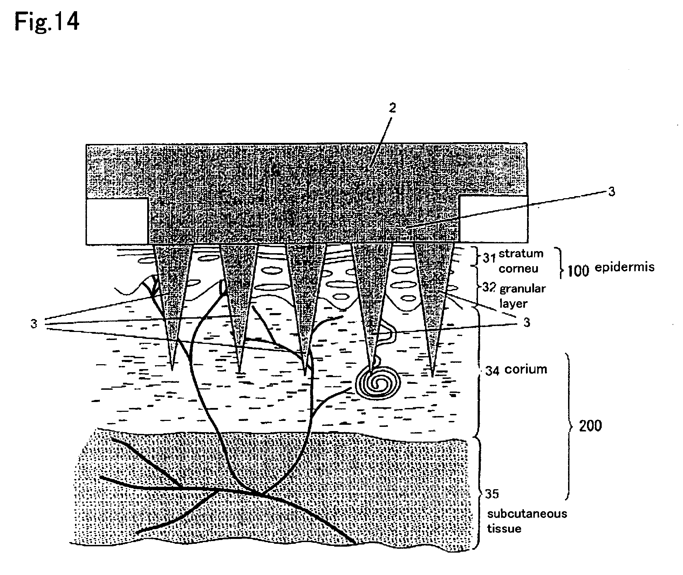

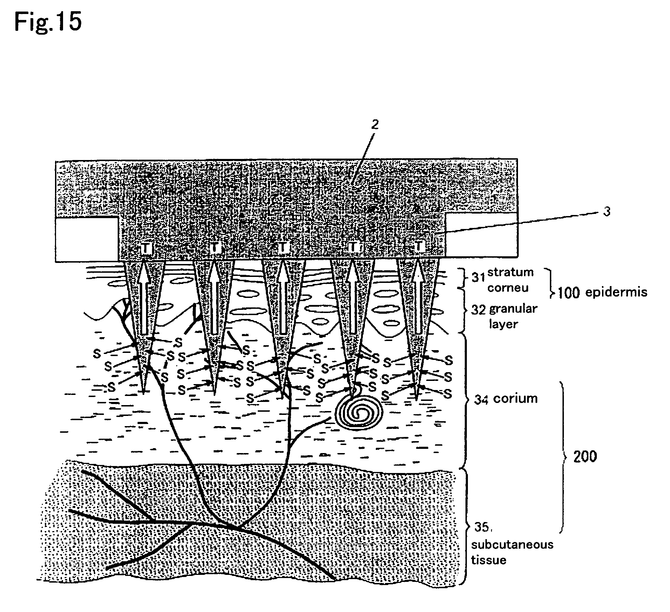

The physiological salt solution 3 brought into contact with the portion A flows into the extraction holes 22a as shown in Fig. 14. When the physiological salt solution 22a flows into the extraction holes 22a, the tissue fluid exuded into the extraction holes 22a as a result of the formation of the extraction holes 22a in the Step S1 moves in the direction of the chamber 2 (T direction shown in Fig.15) as shown in Fig.15. Accordingly, a concentration of the tissue fluid with respect to the physiological salt solution in the extraction holes 22a is lowered. Then, the tissue fluid is extracted into the physiological salt solution 3 in the extraction holes 22a from the corium 34 as shown in arrows S.

When the subject operates the input unit (key switch) 14 to input an instruction for starting the measurement to the controller 10, the electrode 5 is connected to a cathode of the constant voltage power supply 12 and the electrode 6 is connected to an anode of the constant voltage power supply 12 by the switch circuit 13 (Step S3).

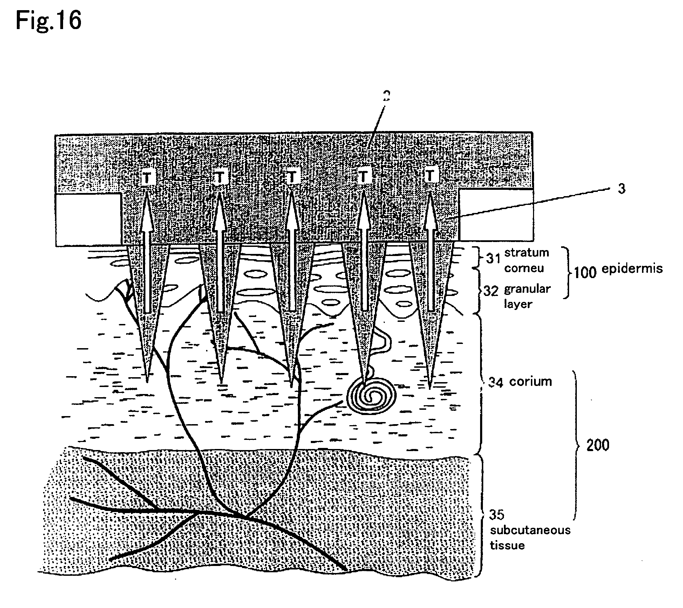

Next, the constant voltage power supply 12 starts to apply the constant voltage of 0.8V to the skin (Step S4) and monitors the current value using the current measuring unit 17 (Fig. 3) (Step S5). Because the tissue fluid exuded into the physiological salt solution in the extraction holes 22a is electrically charged, an electric field imparted by the power supply 12 promotes the movement of the tissue fluid in the direction of the chamber 2 (T direction shown in Fig. 16) as shown in Fig. 16. The glucose included in the tissue fluid is not electrically charged, however, moves along with the movement of other components, which are electrically charged.

In Step S6, the controller 10 acquires a signal outputted from the glucose sensor 4.

In Step S7, the controller 10 judges whether or not the predetermined time (T) has passed from the start of the constant voltage application in the Step S4, while returning to the Step S5 when the predetermined time (T) has not passed yet. More specifically, the constant voltage is continuously applied to the skin, and the monitoring of the current value by the current measuring unit 17 and the acquisition of the signal outputted from the glucose sensor 4 are repeatedly carried out until when the predetermined time (T) has passed from the start of the constant voltage application in the Step S4. The predetermined time (T) is, for example, longer than three minutes and shorter than five minutes

When it is judged in the Step S7 that the predetermined time (T) has passed, the controller 10 calculates the average value Iave of the currents monitored by the current measuring unit 17 until when the predetermined time (T) has passed from the start of the constant voltage application in the Step S8.

In Step S9, the controller 10 calculates the glucose-extracting speed J based on the output signal acquired from the glucose sensor 4 until when the predetermined time (T) has passed from the start of the constant voltage application.

The glucose-extracting speed J is calculated from the following arithmetic expression referring to the glucose amount in the chamber 2 when the predetermined time (T) has passed as Q.

$$J = Q / T \quad \ldots \quad (14)$$

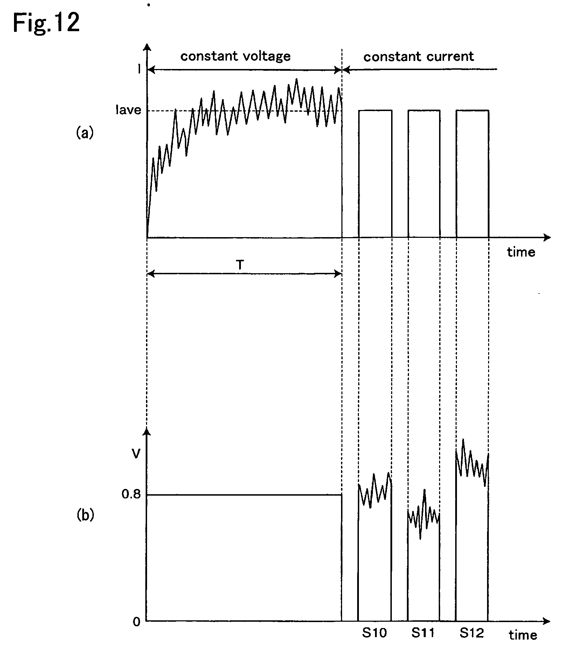

Figs. 12 (a) and (b) are timing charts respectively showing the output currents and the output voltages of the power supplies 11 and 12 from the Steps S4 through S12.

As shown in Fig. 12 (a), the magnitude of the current outputted from the constant voltage power supply 12 changes until when the predetermined time (T) has passed from the start of the constant voltage application, while the value of the voltage outputted from the constant voltage power supply 12 is constant as shown in Fig. 2 (b). As time passes from the start of the constant voltage application, the transmission factor of the epidermis increases, and the current outputted from the constant voltage power supply 12 correspondingly increases by degrees.

Next, in Step S10, the electric resistance Rab between the portions A and B is measured. More specifically, a cathode of the constant current power supply 11 is connected to the electrode 5 and an anode of the constant current power supply 11 is connected to the electrode 6 by the switch circuit 13. Then, the constant current having the same magnitude as that of the average current Iave calculated in the Step S8 is outputted to between the portions A and B by the constant current power supply 11 for ten seconds. During the ten seconds, the voltage measuring unit 16 measures the voltage value between the portions A and B. The controller 10 calculates an average value $Vave_1$ of the voltage values measured by the voltage measuring unit 16, and further calculates the electric resistance value Rab by further dividing $Vave_1$ by Iave.

As shown in the Step S10 in Fig. 12, the magnitude of the current outputted from the constant current power supply 11 is constantly Iave during the ten seconds, while the value of the voltage outputted from the constant current power supply 11 changes.

Next, in Step S11, the electric resistance Rac between the portions A and C is measured. More specifically, the cathode of the constant current power supply 11 is connected to the electrode 7 and the anode of the constant current power supply 11 is connected to the electrode 5 respectively by the switch circuit 13. Then, the constant current having the same magnitude as that of the average current Iave calculated in the Step S8 is outputted to between the portions A and C by the constant current power supply 11 for ten seconds. During the ten seconds, the voltage measuring unit 16 measures the voltage value between the portions A and C. The controller 10 calculates the average value $Vave_2$ of the voltage values measured by the voltage measuring unit 16, and calculates the electric resistance Rac by further dividing $Vave_2$ by Iave.

As shown in the Step 11 in Fig. 12, the magnitude of the current outputted from the constant current power supply 11 is constantly Iave during the ten seconds, while the voltage value outputted from the constant current power supply 11 changes.

Next, in Step S12, the electric resistance Rbc between the portions B and C is measured. More specifically, the cathode of the constant current power supply 11 is connected to the electrode 7 and the anode of the constant current power supply 11 is connected to the electrode 6 by the switch circuit 13. Then, the constant current having the same magnitude as that of the average current Iave calculated in the Step S8 is outputted to between the portions B and C by the constant current power supply 11 for ten seconds. During the ten seconds, the voltage measuring unit 16 measures the voltage value between the portions B and C. The controller 10 calculates an average value $Vave_3$ of the voltage values measured by the voltage measuring unit 16, and calculates the electric resistance Rbc by further dividing $Vave_3$ by Iave.

As shown in the Step 12 in Fig. 12, the magnitude of the current outputted from the constant current power supply 11 is constantly Iave during the ten seconds, while the value of the voltage outputted from the constant current power supply 11 changes.

In Step S13, the controller 10 calculates the electric conductivity k of the portion A of the epidermis using the arithmetic expressions (6) and (7). More specifically, the controller 10 assigns the electric resistance values Rab, Rac and Rbc respectively calculated in the Steps S10 through S12 to the arithmetic expression (6) to calculate the electric resistance value Ra, and assigns the electric resistance value Ra to the arithmetic expression (7) to calculate electric conductivity k.

In Step S14, the controller 10 calculates the blood-sugar level C using the arithmetic expression (12). More specifically, the controller 10 assigns the average current Iave and the electric conductivity k respectively calculated in the Steps S8 and S13 to the arithmetic expression (12) to calculate the blood-sugar level C.

In Step S15, the display unit 15 displays the blood-sugar level calculated in the Step S14.

As thus far described, the blood-sugar level of the subject can be obtained by the controller 10 without the collection of blood for calibration and displayed on the display unit 15.

Therefore, it was conventionally necessary for a diabetic patient to collect blood every day, however, the blood-sugar level measuring device 1 according to the present embodiment eliminates the need to collect blood for the calibration, which favorably alleviates the burden of the subject.

The blood-sugar level measuring device 1 according to the present embodiment extracts the tissue fluid after the preliminary process is performed in the Step S1, however, the present invention is not limited to the procedure. The preliminary step may be omitted so that the voltage is applied to the skin in which the micro holes are not formed in order to extract the glucose (so-called extraction using the reverse iontophoresis method). Further, the preliminary process may be performed with respect to the portion A alone and omitted with respect to the portions B and C.

The blood-sugar level measuring device 1 according to the present embodiment extracts the tissue fluid using the method of applying the current to the skin, however, the present invention is not limited to the method. The Steps S3 through S5 in Fig. 10 may be omitted so that the blood-sugar level measuring device 1 is adapted to extract the tissue fluid using the passive diffusion in which the application of the current to the skin can be omitted.

As the program memorized in the controller10, Iave in the arithmetic expression (12) set to "0" is memorized. As the magnitude of the current supplied in the Steps S10 through S12, a predetermined current value, for example,

30μ A current, may be supplied.

The blood-sugar level measuring device 1 according to the present embodiment uses the direct-current power supply as the constant current power supply 11 and the constant voltage power supply 12, however, the present invention is not limited thereto. The device 1 may be adapted to use an alternating-current power supply as its power supply.

The use of the alternating-current power supply involves such an advantage that the magnitude of the supplied current is stabilized in comparison to the use of the direct-current power supply, which enables the electric resistance value of the epidermis to be accurately calculated.

Further, in the present embodiment, the glucose-extracting speed J is used in the arithmetic expression (2) for obtaining the blood-sugar level C and also in the arithmetic expression for obtaining the transmission factor P, however, the present invention is not limited thereto as far as the value relating to the glucose amount is used. The glucose-extracting speed J in those arithmetic expressions may be replaced with a glucose concentration or a glucose absolute value in the chamber 2.

Further, in the present embodiment, the glucose concentration in the tissue fluid of the body of the subject is regarded as the blood-sugar level, however, the present invention is not limited thereto. The glucose concentration in the tissue fluid in the body of the subject may be converted into the blood-sugar level.

Further, in the present embodiment, the physiological salt solution is supplied from the syringe 2a to the chamber 2, however, the present invention is not limited to the configuration. The blood-sugar level measuring device 1 may be adapted in such manner that a water-retaining member in a dry state (for example, mesh-type nylon sheet) is stored in the chamber 2 and an absorbent cotton containing the physiological salt solution or the like is made to contact the water-retaining member when the blood-sugar level measuring device 1 is used so that the physiological salt solution can be supplied to the chamber 2.

Further, in the present embodiment, the skin resistance is measured (Steps S10 through S12) after the glucose is extracted (Steps S4 through S7), however, the present invention is not limited to the configuration. The blood-sugar level measuring device 1 may be adapted to extract the glucose after the skin resistance is measured. The blood-sugar level measuring device 1 may also be adapted to measure the skin resistance using the current for extracting the glucose.

Further, in the present embodiment, the blood-sugar level measuring device 1 for measuring the blood-sugar level is described as an embodiment of an analyzing device, however, the present invention is not limited thereto. The device 1 can be applied to an analyzer for obtaining a concentration of a component in the tissue fluid in the body of the subject based on an analysis value of the component included in the tissue fluid extracted from the body of the subject. As examples of the concentration of the component analyzable by the analyze according to the present invention, concentrations of a biochemical component, a pharmaceutical product administered to the subject and the like can be mentioned. These components can be extracted into the chamber 2 in the same manner as in the present embodiment. In order to analyze the component extracted into the chamber 2, for example, the ELISA method is used for analyzing protein which is an example of the biochemical component, and the IIPLC method is used for analyzing any biochemical component other than protein and the pharmaceutical product. Then, an analysis result of the component in the chamber 2, which is obtained by means of these analyzing methods, is assigned to "J" in the arithmetic expression (2) so as to obtain a concentration of the component in the tissue fluid in the body of the subject. Further, in the same manner as in the above-mentioned embodiment, the arithmetic expression (9) is assigned to the arithmetic expression (2) as an arithmetic expression for indicating a permeability degree of the component.

Examples of the protein include albumin, globulin, enzyme and the like. Examples of the biochemical component include creatinine, creatine, uric acid, amino acid, fructose, galactose, pentose, glycogen, lactic acid, pyrubic acid, corpus kenton and the like. Examples of the pharmaceutical product include digitalis drug, theophylline, arrhythmia drug, anti-epilepsy drug, amino acid glycoside antibiotic, glycopeptide-based antibiotic, anti-thrombosis drug, immunosuppressive drug and the like.

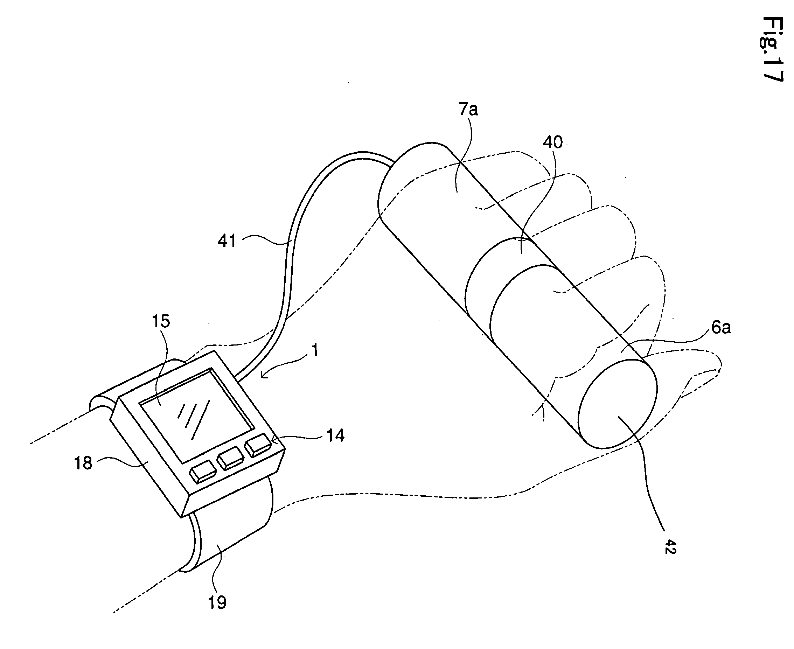

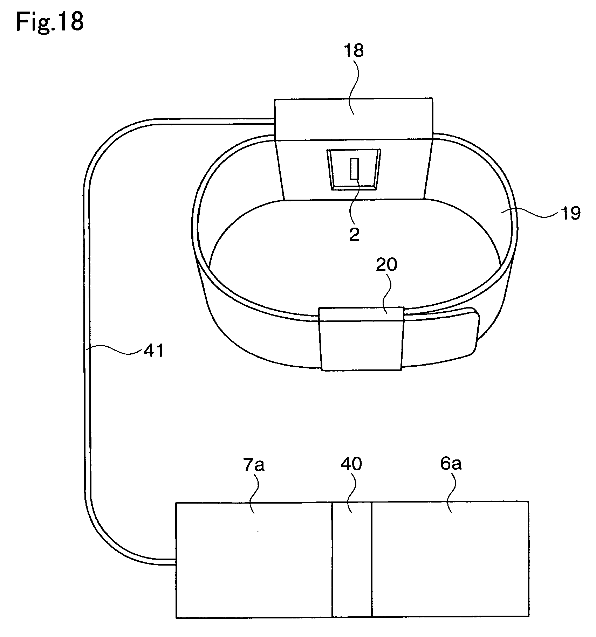

Figs. 17 and 18 show another embodiment corresponding to Figs. 1 and 2.

A blood-sugar level measuring device 1a according to the another embodiment comprises a main body 18a, a band 19, a fixing tool 20 and a electrode bar 42.

The main body 18a has a structure in which the electrode 6 attached to the gelatinous member 8 and the electrode 7 attached to the gelatinous member 9 are eliminated from the main body 18 of the blood-sugar level measuring device 1.

The electrode bar 42 comprises electrodes 6a and 7a as cylindrical members made of aluminum, an insulating member 40 made of polyacetal resin and coaxial with the electrodes 6a and 7a, the insulating member 40 retaining the electrodes 6 and 7 so as to avoid any contact therebetween and a cable 41 formed from two cables bound together. The electrodes 6a and 7a are respectively connected to the switch circuit 13 (see Fig. 3) by the cable 41. The electrode bar 42 is held with a hand of the subject as shown in Fig. 17 so as to contact a palm of the hand when the blood-sugar level is measured, and functions in the same manner as the electrode 6 attached to the gelatinous member 8 and the electrode 7 attached to the gelatinous member 9 in Fig. 3.

The blood-sugar level measuring device 1a, which is constituted as described, does not require the provision of the gelatinous members in the portions B and C. Therefore, it becomes unnecessary to sting the portions B and C of the epidermis using the micro needle array 21.

Further, though it is necessary for the gelatinous members 8 and 9 to be exchanged due to degradation, pollution and the like, the frequency of exchanging the electrodes 6a and 7a is significantly lowered because they are made of metal.

Further, the glucose can be extracted with a smaller voltage (current) because the current circulates through the palm of the hand in contact with the electrodes 6a and 7a more easily than in the case of the skins of the arm and wrist.

**Claims**

1. An analyzer comprising:

   an electrical information acquiring section for acquiring an electrical information by supplying an electricity to a skin of a subject; and
   a controller for acquiring a component value relating to an amount of a predetermined component included in an extraction tissue fluid extracted via the skin of the subject and converting the component value into a concentration of the predetermined component included in an internal tissue fluid existed in a body of the subject based on the electrical information acquired by the electrical information acquiring section.

2. An analyzer according to Claim 1, wherein
   the controller calculates an electric resistance value of a portion of an epidermis through which the extraction tissue fluid passes based on the electrical information and converts the component value into the concentration of the predetermined component included in the internal tissue fluid existed in the body of the subject based on the electric resistance value.

3. An analyzer according to Claim 1, wherein
   the electrical information acquiring section comprises a first electrode, a second electrode, a third electrode, and a power supply connected to the first electrode, the second electrode and the third electrode, and
   the electrical information includes a first electrical information acquired by supplying first current running through the first electrode, the skin and the second electrode by the power supply, a second electrical information acquired by supplying second current running through the first electrode, the skin and the third electrode by the power supply, and a third electrical information acquired by supplying third current running through the second electrode, the skin and the third electrode by the power supply.

4. An analyzer according to Claim 3, wherein
   the power supply supplies fourth current running through the first electrode, the skin and the second electrode to extract the extraction tissue fluid, and
   the controller acquires the component value relating to the amount of the predetermined component included in the extraction tissue fluid extracted by supplying the fourth current by the power supply.

5. An analyzer according to Claim 3, further comprising:

   a retaining unit for retaining the second electrode and the third electrode electrically separated from the second electrode and being held by a hand of the subject in a state where the second electrode and the third electrode are in contact with the skin.

6. An analyzer according to Claim 1, wherein
   the electrical information includes a magnitude of the current running through the skin of the subject.

7. An analyzer according to Claim 1, wherein
   the electrical information includes a magnitude of a voltage applied to the skin of the subject.

8. An analyzer according to Claim 1, further comprising a detector for detecting the predetermined component included in the extraction tissue fluid extracted via the skin of the subject, wherein
   the controller acquires the component value based on a detection result of the detector.

9. An analyzer according to Claim 8, wherein

the controller acquires a speed at which the predetermined component included in the extraction tissue fluid is extracted via the skin as the component value based on the detection result of the detector and a time period required for extracting the predetermined component.

10. An analyzer according to Claim 1, further comprising a tissue fluid retaining material for retaining the extraction tissue fluid.

11. An analyzer according to Claim 2, further comprising a tissue fluid retaining material for retaining the extraction tissue fluid, wherein

the portion of the epidermis through which the extraction tissue fluid passes is a portion in contact with the tissue fluid retaining material.

12. An analyzer according to Claim 1, wherein
the component value is a value relating to a glucose amount, and the concentration of the predetermined component included in the internal tissue fluid existed in the body of the subject is a glucose concentration.

13. An analyzer according to Claim 12, wherein
the glucose concentration is a blood-sugar level of the subject.

14. An analyzer according to Claim 1, further comprising:

a main body comprising the electrical information acquiring section and the controller; and
a fixing member for fixing the main body to the skin of the subject in a state in which the main body is placed on the skin.

15. An analyzing method comprising:

an extracting step for extracting an extraction tissue fluid from a body of a subject via a skin;
a component amount acquiring step for acquiring a component value relating to an amount of a predetermined component included in the extraction tissue fluid extracted in the extracting step;
an electrical information acquiring step for acquiring an electrical information by supplying an electricity to the skin; and
a component concentration acquiring step for converting the component value into a concentration of the predetermined component included in an internal tissue fluid existed in the body of the subject based on the electrical information acquired in the electrical information acquiring step.

16. An analyzing method according to Claim 15, further comprising extraction holes forming step for forming extraction holes penetrating through a stratum corneum but not reaching a subcutaneous tissue in the skin prior to the implementation of the extracting step, wherein
the extraction tissue fluid is extracted via the extraction holes in the extracting step.

17. An analyzing method according to Claim 16, wherein
the extraction tissue fluid is extracted by supplying a liquid in the extraction holes in the extracting step.

18. A blood-sugar level measuring device comprising:

an electrical information acquiring section for acquiring an electrical information by supplying an electricity to a skin of a subject; and
a controller for acquiring a glucose value relating to a glucose amount included in an extraction tissue fluid extracted via the skin of the subject and converting the glucose value into a blood-sugar level of the subject based on the electrical information acquired by the electrical information acquiring section.

19. A blood-sugar level measuring device according to Claim 18, wherein
the controller converts the glucose value into a concentration of the glucose included in an internal tissue fluid existed in a body of the subject based on the electrical information acquired by the electrical information acquiring section, and
the concentration of the glucose included in the internal tissue fluid existed in the body of the subject is the blood-sugar level of the subject.

20. A blood-sugar level measuring device according to Claim 18, wherein
the controller converts the glucose value into a concentration of the glucose included in an internal tissue fluid existed in a body of the subject based on the electrical information acquired by the electrical information acquiring section, and further converts the concentration of the glucose into the blood-sugar level of the subject.

Fig.1

## Fig.2

Fig.3

Fig.4

Fig.5

Fig.6

Fig.7

Fig.8

Fig.9

Fig.10

```
                    ┌─────────┐
                    │  START  │
                    └─────────┘
                         │
                         ▼
        ┌────────────────────────────────┐
        │       preliminary process      │  S1
        └────────────────────────────────┘
                         │
                         ▼
        ┌────────────────────────────────┐
        │          Place device          │  S2
        └────────────────────────────────┘
                         │
                         ▼
        ┌────────────────────────────────┐
        │     set polarity of electrode  │  S3
        └────────────────────────────────┘
                         │
                         ▼
        ┌────────────────────────────────┐
        │ start application of constant voltage │  S4
        └────────────────────────────────┘
                         │
                         ▼◄──────────────────┐
        ┌────────────────────────────────┐   │
        │       monitor current value    │  S5
        └────────────────────────────────┘   │
                         │                    │
                         ▼                    │
        ┌────────────────────────────────┐   │
        │      monitor output of sensor  │  S6
        └────────────────────────────────┘   │
                         │              S7    │
                         ▼                    │
                  ╱───────────────╲           │
                 ╱ passage of time T? ╲───────┘
                  ╲───────────────╱    NO
                    YES  │
                         ▼
        ┌────────────────────────────────┐
        │    calculate average current value │  S8
        └────────────────────────────────┘
                         │
                         ▼
        ┌────────────────────────────────┐
        │  calculate glucose-extracting speed │  S9
        └────────────────────────────────┘
                         │
                         ▼
        ┌────────────────────────────────┐
        │ measure skin resistance between A and B │  S10
        └────────────────────────────────┘
                         │
                         ▼
        ┌────────────────────────────────┐
        │ measure skin resistance between A and C │  S11
        └────────────────────────────────┘
                         │
                         ▼
        ┌────────────────────────────────┐
        │ measure skin resistance between B and C │  S12
        └────────────────────────────────┘
                         │
                         ▼
        ┌────────────────────────────────┐
        │ calculate conductance at extracting portion │  S13
        └────────────────────────────────┘
                         │
                         ▼
        ┌────────────────────────────────┐
        │    calculate blood-sugar level │  S14
        └────────────────────────────────┘
                         │
                         ▼
        ┌────────────────────────────────┐
        │            display             │  S15
        └────────────────────────────────┘
                         │
                         ▼
                    ┌─────────┐
                    │   END   │
                    └─────────┘
```

# Fig.11

Fig.12

Fig.13

# Fig.14

Fig.15

Fig.16

Fig.17

Fig.18

## PARTIAL EUROPEAN SEARCH REPORT

European Patent Office

which under Rule 45 of the European Patent Convention shall be considered, for the purposes of subsequent proceedings, as the European search report

Application Number

EP 05 01 6767

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,L, X | EP 1 552 782 A (SYSMEX CORPORATION) 13 July 2005 (2005-07-13) <br><br> * paragraphs [0022] - [0024] * <br> * paragraphs [0028] - [0043] * <br> * paragraphs [0053] - [0055]; figure 3 * <br> ----- | 1,6-8, 10-14, 18-20 | A61B5/00 A61N1/30 |
| X <br><br> Y | US 6 233 471 B1 (BERNER BRET ET AL) 15 May 2001 (2001-05-15) <br><br><br> * column 11, line 41 - column 13, line 41 * <br> * column 16, lines 7-35 * <br> * column 20, lines 18-35 * <br> * column 28, lines 45-59 * <br> * claims 36-41 * <br> * figures 1A,1B,2,4 * <br> ----- | 1-8, 10-14, 18-20 <br> 9 | |
| D,Y | WO 96/00110 A (CYGNUS THERAPEUTIC SYSTEMS) 4 January 1996 (1996-01-04) <br> * page 15, lines 3-23 * <br> ----- <br><br>-/-- | 9 | |

TECHNICAL FIELDS SEARCHED (Int.Cl.7)

A61B
A61N

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Berlin | 14 October 2005 | Völlinger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

## PARTIAL EUROPEAN SEARCH REPORT

Application Number

EP 05 01 6767

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | US 6 517 482 B1 (ELDEN HARRY RICHARDSON ET AL) 11 February 2003 (2003-02-11)<br><br>* column 3, lines 38-62 *<br>* column 9, line 50 - column 10, line 23 *<br>----- | 1,2,6-8, 10-14, 18-20 | |
| X | EP 1 437 091 A (OLLMAR, STIG; ABERG, PETER; NICANDER, INGRID) 14 July 2004 (2004-07-14)<br>* claims 1,9 *<br>* paragraphs [0010], [0011] *<br>----- | 1-3,5-8, 10-14, 18-20 | |

TECHNICAL FIELDS SEARCHED (Int.Cl.7)

European Patent
Office

**INCOMPLETE SEARCH
SHEET C**

Application Number

EP 05 01 6767

Claim(s) not searched:
     15-17

Reason for the limitation of the search (non-patentable invention(s)):

Article 52 (4) EPC - Method for treatment of the human or animal body by surgery

## ANNEX TO THE EUROPEAN SEARCH REPORT
## ON EUROPEAN PATENT APPLICATION NO.

EP 05 01 6767

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2005

| Patent document cited in search report | | Publication date | Patent family member(s) | | Publication date |
|---|---|---|---|---|---|
| EP 1552782 | A | 13-07-2005 | CN | 1636505 A | 13-07-2005 |
| | | | US | 2005177037 A1 | 11-08-2005 |
| US 6233471 | B1 | 15-05-2001 | US | 2001016682 A1 | 23-08-2001 |
| WO 9600110 | A | 04-01-1996 | AT | 196741 T | 15-10-2000 |
| | | | AU | 2944995 A | 19-01-1996 |
| | | | CA | 2193885 A1 | 04-01-1996 |
| | | | DE | 69519023 D1 | 09-11-2000 |
| | | | DE | 69519023 T2 | 13-06-2001 |
| | | | DK | 766578 T3 | 23-10-2000 |
| | | | EP | 0766578 A1 | 09-04-1997 |
| | | | ES | 2150001 T3 | 16-11-2000 |
| | | | GR | 3034387 T3 | 29-12-2000 |
| | | | JP | 10506293 T | 23-06-1998 |
| | | | JP | 3328290 B2 | 24-09-2002 |
| | | | JP | 2002191582 A | 09-07-2002 |
| | | | PT | 766578 T | 31-01-2001 |
| US 6517482 | B1 | 11-02-2003 | NONE | | |
| EP 1437091 | A | 14-07-2004 | AU | 2003252924 A1 | 29-04-2004 |
| | | | CA | 2444211 A1 | 11-04-2004 |